# EUROPÄISCHE PATENTSCHRIFT

(11) **EP 2 873 386 B1**
(45) Veröffentlichungstag und Bekanntmachung des Hinweises auf die Patenterteilung: **20.03.2019**
(21) Anmeldenummer: 13005414.1
(22) Anmeldetag: 18.11.2013
(51) Int. Cl.: F16M 13/02

(54) **STATIVVORRICHTUNG MIT EINZUGSEINRICHTUNG SOWIE VERFAHREN ZUM EINZIEHEN EINER LEITUNG IN DIE STATIVVORRICHTUNG**
STAND DEVICE WITH RETRACTING DEVICE AND PROCESS FOR PULLING A CONDUIT INTO A STAND DEVICE
DISPOSITIF À PIED DOTÉ D'UN DISPOSITIF D'INSERTION ET PROCÉDÉ D'INSERTION D'UNE LIGNE DANS LE DISPOSITIF À PIED

(43) Veröffentlichungstag der Anmeldung: 20.05.2015
(73) Patentinhaber: Ondal Medical Systems GmbH, 36088 Hünfeld (DE)
(72) Erfinder: Perplies, Stefan, 36088 Hünfeld (DE); Abel, Markus, 36419 Geisa (DE); Volkenand, Kai, 36088 Hünfeld (DE)
(74) Vertreter: Graf von Stosch, Andreas

(56) Entgegenhaltungen:
- EP-A1- 1 473 509
- WO-A1-99/50587
- WO-A1-2008/112675
- DE-U1-202009 014 932
- FR-A- 1 150 794

## Beschreibung

Die vorliegende Erfindung betrifft eine Stativvorrichtung zur Anordnung in einem Operationssaal, die ein Tragsystem und eine medizintechnische Einrichtung umfasst und eingerichtet ist, eine Leitung in die Stativvorrichtung einzuziehen. Die vorliegende Erfindung betrifft ferner ein Verfahren zum Verlegen einer Leitung an einer in einem Operationssaal angeordneten Stativvorrichtung mit den Schritten: Befestigen einer Leitung an einem Zugmittel; Ausüben einer Zugkraft auf das Zugmittel und dadurch Einziehen der Leitung in die Stativvorrichtung mittels des Zugmittels. Die vorliegende Erfindung betrifft insbesondere eine Stativvorrichtung mit einzelnen Merkmalen des Anspruchs 1 sowie ein Verfahren mit einzelnen Merkmalen des unabhängigen Verfahrensanspruchs.

Stative, insbesondere Deckenstative wie z.B. Deckenversorgungseinheiten, weisen meist einen oder mehrere in Bezug auf eine Vertikalposition starr angeordnete oder höhenverstellbare Tragarme auf, mittels welchen eine daran befestigte medizintechnische Einrichtung bewegt und positioniert werden kann, z.B. in einem Operationssaal, insbesondere auch auf einer Intensivstation. An den Stativen sind häufig Versorgungseinheiten montiert, mit denen medizinisch-elektrische Endgeräte mit den z.B. während einer Operation benötigten Medien versorgt werden können. Die Tragarme definieren dabei den Aktionsradius der medizintechnischen Einrichtung.

Zur Versorgung der Endgeräte sind in bzw. an den Tragarmen häufig Leitungen, insbesondere Gas- oder Schlauchleitungen oder Kabelleitungen verlegt. Die Versorgungsleitungen führen üblicherweise von einem Montageflansch am oberen Ende des Stativs zur medizintechnischen Einrichtung, also entlang von einem oder mehreren Tragarmen bzw. entlang von einem oder mehreren zwischen den Tragarmen vorgesehenen Gelenken. Die Versorgungsleitungen können zumindest teilweise bereits bei der Herstellung der Stative bzw. der Versorgungseinheit verlegt werden. In vielen Fällen ist es jedoch erforderlich, bei der Installation des jeweiligen Stativs im Operationssaal oder nachträglich in Hinblick auf einen speziellen Einsatzzweck zusätzliche Schläuche oder Kabel zu verlegen, je nach Auslieferungszustand und je nach Einsatzgebiet. Üblicherweise werden die zusätzlichen Leitungen dann außen an Tragarmen oder in Leerrohren durch die Tragarme geführt.

In manchen Fällen kann werksseitig bereits irgendein Draht oder Seil in einem für eine später einzuziehende Leitung vorgesehenen Leerrohr vormontiert werden. Mit dem Draht kann dann nachträglich noch eine einzelne Leitung auf einfachere Weise durch das Leerrohr hindurchgezogen werden. Mit anderen Worten muss der Draht nicht erst auf mühsame Weise durch das Leerrohr hindurchgefädelt werden. Für ein vollständiges Verlegen der Leitungen müssen jedoch häufig irgendwelche Verkleidungen der Stative demontiert werden, um die Leitungen abschnittsweise entlang einzelner Tragarme daran oder darin verlegen zu können. Bei besonders langen Tragarmen ist diese Art der Montage besonders aufwändig. Insbesondere besteht die Gefahr, dass sich einzelne Leitungen miteinander oder mit irgendwelchen Komponenten des jeweiligen Tragarms verhaken, was zusätzliche Hilfsmittel wie z.B. Zugstangen erfordert. Derlei Probleme treten vor allem dann auf, wenn eine Mehrzahl von Leitungen in demselben Tragarm verlegt werden müssen, insbesondere bei engen Platzverhältnissen.

Ein nachträgliches Verlegen von Leitungen ist daher meist zeitaufwändig und kann unerwartete Schwierigkeiten mit sich bringen, insbesondere aufgrund schlechter Zugänglichkeit. Häufig werden die Stative bzw. Versorgungseinheiten beim nachträglichen Verlegen auch beschädigt, da nur Werkzeuge zur Verfügung stehen, die für diese Arbeiten nicht optimal geeignet sind.

Aus der FR 1 150 794 A ist eine Rückzugshilfe bekannt, welche nur in einer einzigen Richtung wirkt und zwar auf ein bereits verlegtes Kabel. Dabei ist die Rückzugshilfe mit einer Vorspannkraft gelagert, welche insbesondere mittels eines Gewichts und einer Gasfeder einstellbar ist. Die Rückzugshilfe weist zwei Rollen auf, von denen eine beweglich ist.

Es ist eine Aufgabe der vorliegenden Erfindung, das nachträgliche Einziehen von Leitungen, insbesondere Schläuchen oder Kabeln, in Stativvorrichtungen, insbesondere Deckenstativen wie z.B. Deckenversorgungseinheiten, zu erleichtern. Dabei besteht auch ein Interesse an einer kostengünstigen Einrichtung für das nachträgliche Einziehen, welche in einer Stativvorrichtung standardgemäß vorgesehen werden kann.

Die Aufgabe wird durch eine Stativvorrichtung, insbesondere Deckenstativvorrichtung wie z.B. eine Deckenversorgungseinheit, der vorliegenden Erfindung gelöst. Diese Stativvorrichtung zur Anordnung in einem Operationssaal umfasst ein Tragsystem umfassend eine Montageeinrichtung, insbesondere zur Deckenmontage, und mindestens einen Tragarm. Die Stativvorrichtung umfasst ferner eine medizintechnische Einrichtung, die am mindestens einen Tragarm befestigt ist. Die Stativvorrichtung weist eine Einzugseinrichtung auf, die mit einer Leitung verbindbar ist und eingerichtet ist, die Leitung in die Stativvorrichtung, insbesondere in den mindestens einen Tragarm, einzuziehen. Die Einzugseinrichtung ist dabei dazu eingerichtet, die Leitung in einer ersten Richtung oder in einer zweiten Richtung entgegengesetzt zur ersten Richtung einzuziehen und zu verlegen.

Mittels der Einzugseinrichtung kann eine beliebige Person nachträglich auf einfache Weise irgendwelche Leitungen, insbesondere Schläuche oder Kabel, in einen jeweiligen Tragarm einziehen, insbesondere ohne Spezialwerkzeug. Eine Beschädigung des jeweiligen Tragarms kann dabei vermieden werden. Auch kann die erforderliche Montagezeit bzw. Installationszeit verringert werden. Die Einzugseinrichtung kann z.B. an der Stativvorrichtung vormontiert sein oder bei bestehenden, bereits montierten Stativvorrichtung nachgerüstet werden.

Als medizintechnische Einrichtung ist dabei bevorzugt eine Versorgungskonsole zu verstehen, mittels welcher Mittel für eine Versorgung eines Patienten und/oder Instrumente für einen Operateur und/oder Licht, Reinluft oder andere im Operationssaal benötigte Medien bereitgestellt werden können. Die medizintechnische Einrichtung weist bevorzugt irgendein Bedienpanel und/oder irgendeine Anzeigevorrichtung zum grafischen Darstellen von z.B. Patientendaten auf.

Als Montageeinrichtung ist dabei bevorzugt ein Flansch oder irgendeine andere Schnittstelle zu verstehen, womit das Tragsystem an einer zumindest annähernd horizontal ausgerichteten Zimmerdecke oder auch einer zumindest annähernd vertikal ausgerichteten Wand montiert werden kann. Mit anderen Worten betrifft die vorliegende Erfindung auch Stativvorrichtungen, welche an vertikalen Wänden montiert werden können. Auch eine an einer vertikalen Wand gelagerte medizintechnische Einrichtung kann Teil der erfindungsgemäßen Stativvorrichtung sein.

Als Tragarm ist dabei bevorzugt ein Ausleger oder Träger zu verstehen, welcher sich in einer bestimmten Richtung erstreckt und den gewünschten Aktionsradius für die unterschiedlichen Soll-Positionen der medizintechnischen Einrichtung sicherstellen kann, insbesondere durch eine Drehbewegung um ein Drehgelenk. Der Tragarm kann auch eine teleskopische Vorrichtung mit einem (zusätzlichen) Bewegungsfreiheitsgrad in translatorischer Richtung entlang der Längsachse des Tragarms sein.

Als Einzugseinrichtung ist dabei bevorzugt eine Einrichtung zu verstehen, die eine formstabile Komponente und mindestens eine relativ zur formstabilen Komponente verlagerbare Kupplung für die Leitung aufweist, wobei die formstabile Komponente mit der Stativvorrichtung fest verbunden werden kann und ortsfest an der Stativvorrichtung angeordnet werden kann. Die formstabile Komponente kann z.B. eine starre Platte sein, an welcher eine Bohrung oder eine Klebestelle zum Befestigen der Platte an der Stativvorrichtung, insbesondere an einer Innenseite eines Tragarms, vorgesehen ist. Die Kupplung kann z.B. über ein Seil mit der Platte verbunden sein.

Als Leitung ist dabei bevorzugt jede Art von elektrischem Kabel oder jede Art von Schlauch zu verstehen. Der Schlauch kann irgendein Fluid führen, sei es ein Gas oder eine Flüssigkeit. Die Leitung kann z.B. aus einem Kunststoffmaterial oder zumindest teilweise aus einem metallischen Material bestehen.

Bevorzugt weist die Einzugseinrichtung eine Montageschnittstelle auf, mittels welcher die Einzugseinrichtung ortsfest an der Stativvorrichtung fixierbar ist. Die Montageschnittstelle kann formschlüssig, kraftschlüssig und/oder stoffschlüssig ausgebildet sein, z.B. als Bohrung, als adhäsive Fläche oder irgendeine andere formschlüssige Schnittstelle. Hierdurch kann sichergestellt werden, dass mittels der Einzugseinrichtung eine Kraft auf die Leitung ausgeübt werden kann, ohne die Einzugseinrichtung relativ zur Stativvorrichtung komplett verlagern zu müssen.

Gemäß einem Ausführungsbeispiel ist die Einzugseinrichtung am mindestens einen Tragarm befestigt. Hierdurch kann die Leitung relativ zum Tragarm auf genaue Weise positioniert werden, und die auf die Leitung ausgeübte Kraft kann in den Tragarm weitergeleitet werden. Hierbei kann auch eine Umlenkung erfolgen, so dass eine Zugrichtung zum Einziehen der Leitung nicht notwendigerweise der Richtung entsprechen muss, in welche die Leitung eingezogen werden soll.

Gemäß einem Ausführungsbeispiel ist die Einzugseinrichtung innerhalb vom mindestens einen Tragarm angeordnet, insbesondere an einer Oberseite des Tragarms. Hierdurch kann die Leitung mittels der Einzugseinrichtung in diejenige Soll-Position gezogen werden, in welcher die Leitung auch innerhalb vom Tragarm befestigt werden soll. Eine Anordnung kann an einer der Innenseiten des Tragarms erfolgen, z.B. an einer Oberseite, einer Unterseite oder an insbesondere in horizontale Richtung weisenden Seitenflächen. Eine Anordnung an der Oberseite des Tragarms hat den Vorteil, dass auf der Unterseite weiterhin Platz für üblicherweise an der Unterseite angeordnete Komponenten, z.B. eine Parallelführung, vorhanden ist. Bei einer Anordnung an der Oberseite kann die Einzugseinrichtung daher ohne aufwändige Änderungen vorgesehen werden, z.B. auch nachgerüstet werden. Eine Anordnung an der Oberseite des Tragarms hat auch den Vorteil, dass einzelne Komponenten der Einzugseinrichtung aufgrund der Schwerkraft von der Oberseite beabstandet sind, so dass weitere an der Oberseite angeordnete Komponenten nicht von irgendwelchen Komponenten der Einzugseinrichtung behindert werden.

Gemäß einem Ausführungsbeispiel weist die Einzugseinrichtung eine Platte auf, insbesondere ein metallisches Strangpressprofil oder Kunststoffteil. Hierdurch kann zum einen eine Anlagefläche bereitgestellt werden, mittels welcher die Einzugseinrichtung am Tragarm abgestützt werden kann. Zum anderen kann die Einzugseinrichtung durch ein kostengünstiges Standard-Profil mit einer guten Steifigkeit gebildet werden. Als Platte ist dabei bevorzugt ein starres Teil zu verstehen, auf welchem einzelne Komponenten der Einzugsanordnung relativ zueinander in einer vorbestimmten Anordnung anordenbar sind.

Gemäß einem Ausführungsbeispiel erstreckt sich die Einzugseinrichtung entlang des mindestens einen Tragarms und ist derart daran angeordnet, dass die Einzugseinrichtung sowohl von einem ersten Ende des Tragarms als auch von einem zweiten Ende des Tragarms für eine manuelle Betätigung der Einzugseinrichtung zugänglich ist. Hierdurch kann eine Leitung wahlweise vom ersten Ende oder vom zweiten Ende in den Tragarm eingezogen werden.

Bevorzugt weist die Einzugseinrichtung eine Länge auf, welche mindestens im Bereich von 50 Prozent der Länge des Tragarms liegt, an welchem die Einzugseinrichtung angeordnet ist. Die Länge der Einzugseinrichtung kann dabei durch den Abstand von zwei gegenüberliegenden Enden der Einzugseinrichtung bestimmt werden. Insbesondere kann die Länge auch durch den Verlagerungsweg, welcher mittels der Einzugseinrichtung ermöglicht wird, definiert werden. Für diesen Fall kann die Länge der Einzugseinrichtung auch z.B. über den Abstand von zwei Umlenkeinheiten zueinander definiert werden. Weiter bevorzugt liegt die Länge der Einzugseinrichtung im Bereich von 60 bis 90 Prozent der Länge des Tragarms, besonders bevorzugt im Bereich von 70 bis 80 Prozent der Länge des Tragarms. Hierdurch kann eine leichte Zugänglichkeit zu beiden Enden der Einzugseinrichtung sichergestellt werden.

Gemäß einer Variante ist die Einzugseinrichtung mittig in Bezug auf die beiden Ende des Tragarms am Tragarm angeordnet. Bevorzugt weist die Einzugseinrichtung dabei eine Länge auf, welche maximal 75 Prozent der Länge des Tragarms beträgt. Hierdurch kann Material eingespart werden, und es kann eine besonders kostengünstige Einzugseinrichtung bereitgestellt werden.

Gemäß einem Ausführungsbeispiel ist die Einzugseinrichtung eingerichtet, die Leitung in einer ersten Richtung oder in einer zweiten Richtung entgegengesetzt zur ersten Richtung einzuziehen. Hierdurch kann ein nachträgliches Einziehen einer Leitung an einem Ende eines Tragarms erfolgen, welches besonders gut zugänglich ist. Auch kann die Leitung entweder von der Montageeinrichtung (insbesondere einem Deckenflansch) zur Konsole oder in entgegengesetzter Richtung verlegt werden, je nachdem welche Richtung vorteilhafter ist. Mit anderen Worten ist irgendeine Kupplung der Einzugseinrichtung zum Verbinden mit der Leitung bidirektional verlagerbar, insbesondere bidirektional manuell betätigbar.

Gemäß einem Ausführungsbeispiel weist die Stativvorrichtung eine Mehrzahl von Tragarmen auf, wobei innerhalb von mindestens zwei der Tragarme jeweils eine Einzugseinrichtung angeordnet ist. Hierdurch kann eine Leitung auf einfache Weise auch entlang von mehreren Tragarmen, insbesondere komplett vom Deckenflansch bis zur Konsole verlegt werden.

Gemäß einer Variante weist die Einzugseinrichtung eine Grundplatte (und wahlweise auch davon abstehende Seitenwände) auf, die aus einem Strangpressprofil, insbesondere einem Aluminiumstrangpressprofil gebildet ist. Gemäß einer Variante weist die Einzugseinrichtung eine starre Komponente in Form eines U-Profils auf, wobei eine Seite des U-Profils durch die Grundplatte gebildet sein kann, und gegenüberliegende Seiten des U-Profils können durch die Seitenwände gebildet sein. Ein U-Profil kann kostengünstig hergestellt werden und kann die einzelnen Komponenten der Einzugseinrichtung (insbesondere ein Zugmittel und Befestigungseinheiten) derart aufnehmen, dass sie innerhalb der Einzugseinrichtung angeordnet bleiben. Hierdurch kann sichergestellt werden, dass weitere Komponenten der Stativvorrichtung nicht behindert werden.

Gemäß einem Ausführungsbeispiel weist die Einzugseinrichtung ein Zugmittel zum Aufbringen einer Zugkraft auf die Leitung auf. Hierdurch kann die Leitung von einem ersten Ende des Tragarms durch den Tragarm gezogen werden, indem eine Kraft von einem zweiten Ende des Tragarms auf die Leitung ausgeübt wird. Eine Zugkraft kann dabei unabhängig von der Steifigkeit der Leitung das Einziehen der Leitung sicherstellen, auch bei einer bedeutenden Länge des Tragarms, z.B. bei über einem Meter, oder sogar über zwei oder mehr Metern Länge. Auch kann ein Zugmittel besonders kostengünstig und gewichtsoptimiert ausgeführt werden. Ein Zugmittel kann auch auf einfache Weise umgelenkt werden.

Als Zugmittel ist dabei bevorzugt eine in sich flexible Einrichtung zu verstehen, welches eine Zugkraft übertragen kann. Das Zugmittel kann z.B. zumindest teilweise durch ein Seil, Tau, Strick, oder durch ein Band, eine Schnur oder eine Kordel gebildet sein. Bevorzugt ist das Zugmittel durch ein Seil gebildet, insbesondere ein Drahtseil wie z.B. ein Stahlseil. Das Zugmittel kann auch durch ein Seil aus einer Textilfaser oder einem Kunststoff bzw. einer Kunststofffaser wie z.B. einer synthetischen Chemiefaser auf der Basis von Polyethylen (z.B so genanntes Dyneema) gebildet sein. Ein Stahlseil oder ein Tau aus Dyneema kann eine große Zugkraft übertragen und weisen dabei nur einen kleinen Durchmesser auf, nimmt also nur wenig Platz innerhalb vom Tragarm ein.

Gemäß einem Ausführungsbeispiel weist das Zugmittel mindestens eine manuelle Betätigungseinheit auf, insbesondere einen formbeständigen Griff. Hierdurch kann das Zugmittel auf einfache Weise händisch bedient werden, also ohne Spezialwerkzeug. Dies erleichtert die Montage, und die Stativvorrichtung wird nicht durch Zangen oder dergleichen massives Werkzeug beschädigt. Die Betätigungseinheit kann dabei auch aus einem Tragarm hervorragen, so dass die Zugänglichkeit und Montage für einen Monteur weiter erleichtert wird.

Als manuelle Betätigungseinheit ist dabei bevorzugt irgendeine ergonomische Schnittstelle zu verstehen, welche das Aufbringen einer Zugkraft auf das Zugmittel erleichtert. Bei einem als Seil ausgebildeten Zugmittel kann bereits ein Knoten auf dem Seil die Betätigung erleichtern. Bevorzugt weist die Betätigungseinheit eine Anlagefläche für mehrere Finger einer menschlichen Hand auf, so dass eine Zugkraft ohne Druckstellen bzw. Druckspitzen an der Hand zwischen dem Zugmittel und der Hand übertragen werden kann.

Gemäß einem Ausführungsbeispiel weist die Einzugseinrichtung mindestens einen Rückstellmechanismus, insbesondere einen Kraftspeicher, zum automatischen Beaufschlagen des Zugmittels mit einer Kraft, insbesondere einer Zugkraft, in Reaktion auf eine Verlagerung des Zumittels auf. Hierdurch können mehrere Leitungen auf einfache Weise nacheinander mit derselben Einzugseinrichtung ausgehend von derselben Ausgangslage verlegt werden. Der Rückstellmechanismus kann sicherstellen, dass eine Kupplung zum Verbinden der Einzugseinrichtung mit der Leitung nach dem Einziehen einer ersten Leitung wieder manuell zugänglich ist, insbesondere wieder zu einem Ende des Tragarms zurückverlagert wird. Dabei kann eine Gegenkraft auf das Zugmittel ausgeübt werden, insbesondere um das Zugmittel zurück in eine Ausgangslage (Null-Position) zu verlagern, nachdem die Leitung in eine Soll-Position gebracht wurde.

Als Rückstellmechanismus ist dabei bevorzugt eine Einrichtung zu verstehen, welche automatisch in Reaktion auf eine Kraft in einer ersten Richtung eine Reaktionskraft in einer zweiten Richtung, bevorzugt entgegengesetzt zur ersten Richtung, ausüben kann.

Als Kraftspeicher ist dabei bevorzugt eine elastische Feder, insbesondere eine Konstantfeder zu verstehen, wobei der Kraftspeicher auch als ein Energiespeicher bezeichnet werden kann. Der Kraftspeicher kann jede Form eines Speichers sein, in welchem eine zum Rückstellen des Zugmittels erforderliche Kraft oder Energie bereitgestellt werden kann. Die Feder kann z.B. eine Spiralfeder sein, insbesondere da die Spiralfeder einen großen Federweg ermöglicht. Wahlweise kann z.B. auch eine andere Art elastische Feder oder eine Gasdruckfeder als Kraftspeicher verwendet werden. Wahlweise können auch mehrere Federn zusammengeschaltet sein, insbesondere in Serie, speziell bei besonders langen Tragarmen bzw. besonders langen Einzugseinrichtungen. Bevorzugt ist der Kraftspeicher als Konstantkraftfeder ausgebildet. Hierdurch kann auch bei einem langen Federweg eine konstante (Rückstell-)Kraft auf das Zugmittel ausgeübt werden. Dies erleichtert die Handhabung, und das Zugmittel wird nicht mit immer größer werdender Kraft vorgespannt. Ein Bediener braucht dann auch mit zunehmendem Verlagerungsweg keine größere Kraft aufbringen. Als Konstant(kraft)feder ist dabei eine elastisch verformbare Feder zu verstehen, welche über zumindest einem Abschnitt des Federwegs eine zumindest annähernd konstante Kraft aufbringen kann. Bevorzugt ist der Kraftspeicher mit einem Ende des Zugmittels verbunden.

Gemäß einem Ausführungsbeispiel umfasst die Einzugseinrichtung mindestens eine Umlenkeinheit, insbesondere Umlenkrolle, zum Umlenken des Zugmittels. Hierdurch kann eine Zugkraft auf die Leitung von einem gut zugänglichen Zugangspunkt ausgeübt werden, also ohne dass die Einzugseinrichtung oder der entsprechende Tragarm von der Seite her zugänglich sein muss, zu welcher die Leitung gezogen werden soll.

Als Umlenkeinheit ist dabei bevorzugt jede Einheit zu verstehen, welche ein Umlenken des Zugmittels ermöglicht, insbesondere in einem Winkel von zumindest annähernd 180°. Bei einem Winkel von 180° kann die Wirkungsrichtung umgekehrt werden. Mit anderen Worten kann die Umlenkeinheit sicherstellen, dass die Leitung in einer Richtung entgegensetzt zur Zugrichtung in den Tragarm eingezogen werden kann.

Die Umlenkeinheit ist bevorzugt als drehbar gelagerte Umlenkrolle oder feststehender Umlenkbolzen ausgebildet. Die Umlenkeinheit kann z.B. aus einem metallischen Material, insbesondere Stahl, oder aus einem Kunststoff gefertigt sein. Ein feststehender Umlenkbolzen kann eine besonders kostengünstige Ausgestaltung der Einzugseinrichtung ermöglichen. Ein Drehlager ist nicht erforderlich. Die Einzugseinrichtung kann besonders wartungsarm ausgeführt werden. Auch kann verhindert werden, dass sich das Zugmittel verklemmt oder ein Lager der Umlenkeinheit blockiert. Die Umlenkeinheit kann aus einem Material mit hoher Festigkeit und geringem Reibungswiderstand ausgeführt sein oder eine entsprechende Beschichtung aufweisen. Bevorzugt ist die Umlenkeinheit zumindest abschnittsweise aus Polyoxymethylen (POM) ausgeführt oder weist eine Beschichtung aus Polyoxymethylen (POM) auf. Es hat sich gezeigt, dass Polyoxymethylen (POM) eine ausreichend hohe Festigkeit liefert und einen niedrigen Reibungskoeffizienten aufweist.

Gemäß einem Ausführungsbeispiel weist die Einzugseinrichtung eine Befestigungseinheit zum Befestigen einer Leitung an der Einzugseinrichtung, insbesondere an einem Zugmittel der Einzugseinrichtung, auf. Hierdurch kann die Befestigung der Leitung mit dem Zugmittel auf einfachere Weise erfolgen. Insbesondere kann das Material der Befestigungseinheit flexibler (weniger starr) sein als dasjenige des Zugmittels. Eine Befestigungseinheit kann auch sicherstellen, dass das Zugmittel in Verbindung mit mehreren freien Enden bereitgestellt werden kann. Mehrere freie Enden können die Bedienung der Einzugseinrichtung von mehreren Seiten sicherstellen.

Als Befestigungseinheit ist dabei bevorzugt irgendeine Kupplung zu verstehen, an welcher eine Leitung reversibel befestigt werden kann, sei es durch Formschluss und/oder durch Kraftschluss. Gemäß einer Variante ist die Befestigungseinheit zumindest teilweise durch das Zugmittel gebildet.

Bevorzugt ist die Befestigungseinheit an einem vordefinierten Befestigungspunkt mit dem Zugmittel verbunden, wobei der Befestigungspunkt derart relativ zum Zugmittel angeordnet ist, dass ein großer Verlagerungsweg realisiert werden kann. Der Befestigungspunkt ist (in Bezug auf eine Ausgangsposition des Zugmittels) bevorzugt im Bereich der Umlenkeinheit(en) bzw. im Bereich eines ersten oder zweiten Endes der Einzugseinrichtung angeordnet.

Bevorzugt weist die Einzugseinrichtung drei freie Enden auf. Bevorzugt sind zwei der drei freien Enden durch jeweils eine Befestigungseinheit gebildet, und das weitere freie Ende ist durch eine Betätigungseinheit gebildet. Hierdurch kann eine Betätigung immer in derselben Richtung bzw. immer am gleichen Ende der Einzugseinrichtung erfolgen, und zwar unabhängig davon, in welcher Richtung eine Leitung entlang der Einzugseinrichtung verlagert werden soll.

Bevorzugt weist die Einzugseinrichtung mindestens zwei Umlenkeinheiten sowie eine im Bereich jeweils einer der Umlenkeinheiten angeordnete Befestigungseinheit auf. Hierdurch kann ein langer Verlagerungsweg bereitgestellt werden, und die Befestigungseinheiten können mittels des Rückstellmechanismus wieder an einen gut zugänglichen Punkt (insbesondere bis hin zur Umlenkeinheit) zurückverlagert werden. Die jeweilige Befestigungseinheit kann im Bereich der Umlenkeinheit mit dem Zugmittel verbunden sein. Bevorzugt ist eine der beiden Befestigungseinheiten an einem Ende des Zugmittels angeordnet, und die andere der beiden Befestigungseinheit im Bereich einer der beiden Umlenkeinheiten. Die andere Befestigungseinheit ist bevorzugt ortsfest auf dem Zugmittel fixiert. Die Befestigungseinheiten sind bevorzugt über ein flexibles Stück Seil oder Tau, insbesondere aus einer besonders flexiblen Textilfaser, mit dem Zugmittel verbunden. Hierdurch kann sichergestellt werden, dass die Befestigungseinheiten widerstandsarm zurückverlagert werden können.

Bevorzugt sind die Umlenkeinheiten jeweils an einem ersten und zweiten Ende der Einzugseinrichtung, insbesondere einer Grundplatte der Einzugseinrichtung, angeordnet. Hierdurch kann eine große Längenänderung sicherstellt werden. Mit anderen Worten sind die Umlenkeinheiten in einem möglichst großen Abstand voneinander angeordnet, so dass eine Leitung besonders weit in einen Tragarm eingezogen werden kann.

Bevorzugt weist die Befestigungseinheit eine Schlaufe auf. Hierdurch kann eine Leitung auf einfache Weise ohne weiteres Werkzeug mit dem Zugmittel verbunden werden. Eine Schlaufe kann leicht über ein freies Ende der Leitung gelegt werden und z.B. reibschlüssig an der Leitung zur Anlage kommen. Es kann z.B. eine Schlaufe verwendet werden, welche sich selbst zusammenzieht und die Leitung einklemmt.

Gemäß einer Variante ist die Schlaufe durch das Zugmittel gebildet, insbesondere durch ' eine mit einer Seilendhülse fixierte Bucht mit einem definierten Durchmesser. Die Seilendhülse kann z.B. irreversibel auf das Zugmittel aufgepresst sein. Die Schlaufe kann also ohne Knoten oder andere Arten von Befestigungen, die sich möglicherweise wieder lösen könnten, durch das Zugmittel selbst gebildet sein. Hierdurch kann eine robuste und einfach bzw. kostengünstig ausgebildete Einzugseinrichtung bereitgestellt werden.

Gemäß einem Ausführungsbeispiel weist die Einzugseinrichtung eine manuelle Betätigungseinheit auf, welche an einem Zugmittel der Einzugseinrichtung fixiert ist, wobei das Zugmittel zwei Befestigungseinheiten bereitstellt, von denen die eine Befestigungseinheit von einem ersten Ende der Einzugseinrichtung hervorsteht und von denen die andere Befestigungseinheit von einem zweiten Ende der Einzugseinrichtung hervorsteht. Bei dieser Ausgestaltung kann eine Leitung wahlweise von einem der beiden Enden der Einzugseinrichtung auf ergonomische Weise mit dem Zugmittel verbunden werden bzw. wieder vom Zugmittel gelöst werden.

Damit die Befestigungseinrichtung hervorsteht und gut zugänglich ist, kann sie einen Verlängerungsabschnitt aufweisen. Mit anderen Worten kann die Befestigungseinheit einen Verlängerungsabschnitt umfassen, mittels dessen sichergestellt werden kann, dass eine Leitung auch in einem gewissen Abstand von einer Umlenkeinheit mit dem Zugmittel verbunden werden kann. Der Verlängerungsabschnitt kann auch sicherstellen, dass die Leitung, nachdem sie durch einen Tragarm hindurchgezogen wurde, auf ergonomische Weise wieder vom Zugmittel entkoppelt werden kann.

Die zuvor genannte Aufgabe wird auch bereits durch die zuvor beschriebene Einzugseinrichtung für die Stativvorrichtung gelöst, insbesondere durch eine Einzugseinrichtung, die eine Platte sowie ein relativ zur Platte verlagerbares und mit der Platte verbundenes Zugmittel aufweist, welches mit einer Leitung zum Einziehen der Leitung in die Stativvorrichtung verbindbar ist. Die Einzugseinrichtung ist eingerichtet, ortsfest an der Stativvorrichtung montiert zu werden. Hierzu weist die Einzugseinrichtung eine Montageschnittstelle auf, insbesondere in Form einer Bohrung oder einer adhäsiven Fläche. Die Einzugseinrichtung weist bevorzugt eine Längenabmessung auf, welche an die Längenabmessung eines (jeweiligen) Tragarms der Stativvorrichtung angepasst ist. Bevorzugt weist die Einzugseinrichtung eine Länge auf, welche mindestens im Bereich von 50 Prozent der Länge des Tragarms liegt, an welchem die Einzugseinrichtung angeordnet ist.

Die zuvor genannte Aufgabe wird auch durch ein Verfahren mit den Merkmalen des unabhängigen Verfahrensanspruchs gelöst. Dieses Verfahren zum Verlegen einer Leitung an einer in einem Operationssaal angeordneten Stativvorrichtung, insbesondere einer erfindungsgemäßen Stativvorrichtung, ist durch die folgenden Schritte gekennzeichnet:
- Befestigen einer Einzugseinrichtung an der Stativvorrichtung;
- Befestigen einer Leitung an einem Zugmittel der Einzugseinrichtung;
- Ausüben einer Zugkraft auf das Zugmittel zum Verlagern der Leitung mittels des Zugmittels relativ zur Stativvorrichtung, wobei das Zugmittel von einer Ausgangsposition verlagert wird; und dadurch
- Einziehen der Leitung in die Stativvorrichtung mittels des Zugmittels. Bei diesem Verfahren ergeben sich die zuvor im Zusammenhang mit der Stativvorrichtung beschriebenen Vorteile.

Wahlweise kann das Verfahren ferner den folgenden Schritt umfassen: Zurückverlagern des Zugmittels in die Ausgangsposition, insbesondere mittels eines Rückstellmechanismus.

In den nachfolgenden Zeichnungsfiguren wird die Erfindung anhand von Ausführungsbeispielen näher erläutert. Es zeigen:
- Figur 1: in schematischer Darstellung in perspektivischer Ansicht von unten eine Stativvorrichtung gemäß einem Ausführungsbeispiel der Erfindung;
- Figur 2: in einer Seitenansicht und in einer Draufsicht eine technische Zeichnung einer Einzugseinrichtung für eine Stativvorrichtung gemäß einem Ausführungsbeispiel der Erfindung;
- Figur 3: in schematischer Darstellung in einer perspektivischen Ansicht die in der Fig. 2 gezeigte Einzugseinrichtung;
- Figur 4: in schematischer Darstellung in einer perspektivischen Seitenansicht einzelne Komponenten der in der Fig. 2 gezeigten Stativvorrichtung;
- Figur 5: in schematischer Darstellung in einer perspektivischen Seitenansicht eine Stativvorrichtung gemäß einem weiteren Ausführungsbeispiel; und
- Figur 6: in schematischer Darstellung Verfahrensschritte eines Verfahrens gemäß einem Ausführungsbeispiel der Erfindung.

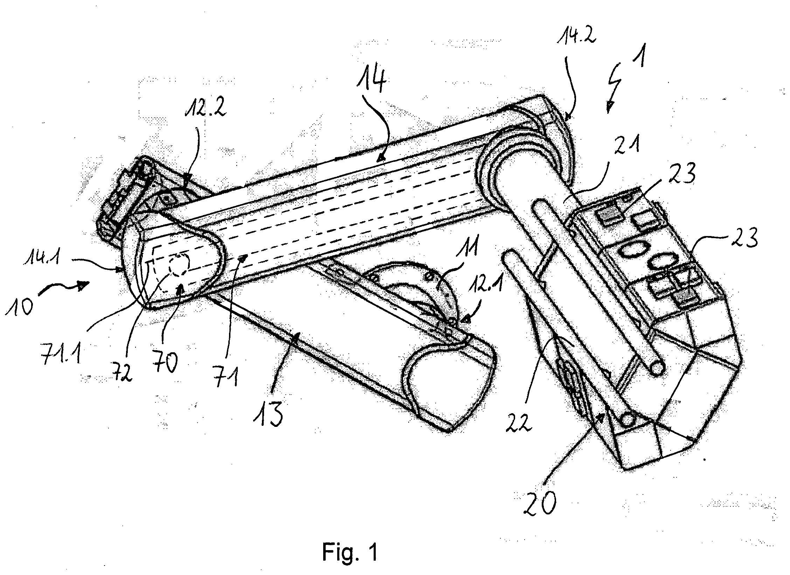

In der Figur 1 ist eine Stativvorrichtung 1 gezeigt, welche ein Tragsystem 10 umfasst, das mittels einer Montageeinrichtung, insbesondere einem Deckenflansch, an einer Decke oder Wand eines Operationssaals montiert werden kann. Das Tragsystem 10 weist einen ersten Tragarm 13 und einen zweiten Tragarm 14 auf, welche jeweils über ein Gelenk 12.1, 12.2 schwenkbar gelagert sind. Am zweiten Tragarm 14 ist eine medizintechnische Einrichtung, insbesondere Versorgungskonsole 20, befestigt, nämlich über den Träger 21, wobei an der Konsole 20 Griffe 22 und Tasten 23 für eine Bedienung der Konsole angeordnet sind. Im zweiten Tragarm 14 ist eine Einzugseinrichtung 70 angeordnet, welche sich innerhalb des Tragarms von einem ersten Ende 14.1 des Tragarms bis zu einem zweiten Ende 14.2 erstreckt. Die Einzugseinrichtung 70 weist ein Zugmittel 71 auf, welches sich zumindest annähernd entlang der gesamten Längserstreckung der Einzugseinrichtung 70 erstreckt und an einer Umlenkeinheit 72 umgelenkt ist. Das Zugmittel 71, insbesondere als Seil ausgeführt, überragt ein erstes Ende 70.1 der Einzugseinrichtung, steht also vom ersten Ende 70.1 hervor. In diesem Bereich weist das Zugmittel eine Kupplung, insbesondere Befestigungseinheit 71.1, auf, an welcher eine Leitung (nicht dargestellt) mit dem Zugmittel 71 verbunden werden kann. Die Befestigungseinheit 71.1 kann z.B. als Schlaufe ausgebildet sein und durch das Zugmittel 71 selbst gebildet sein, also z.B. als eine Drahtschlaufe.

Die Leitung kann mit der Kupplung 71.1 am Ende 14.1 verbunden werden, um die Leitung daraufhin mittels des Zugmittels 71 durch den zweiten Tragarm 14 zu ziehen, insbesondere bis hin zum zweiten Ende 14.2.

Im Zusammenhang mit der Beschreibung der folgenden Figuren wird bei Bezugszeichen, die nicht explizit erläutert werden, auf die weiteren Figuren verwiesen.

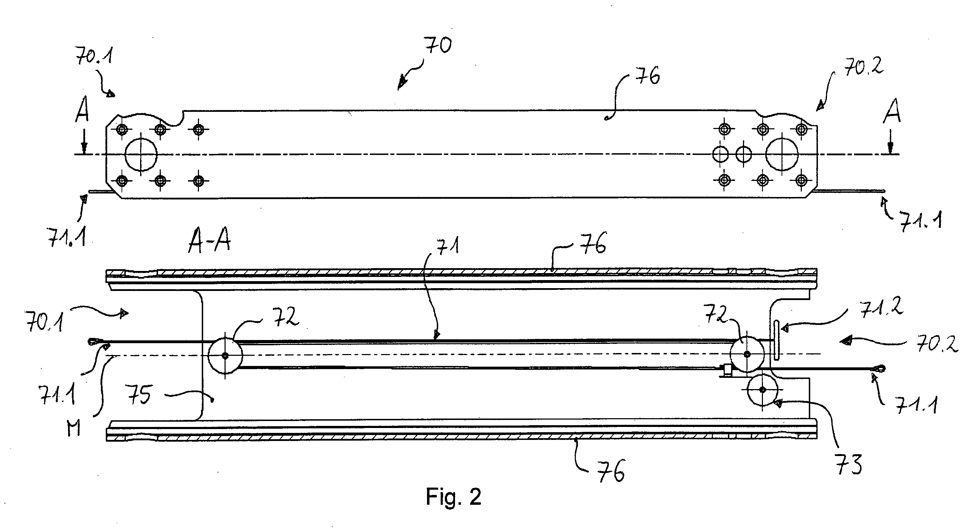

In der Figur 2 ist eine Einzugseinrichtung 70 gezeigt, welche eine Grundplatte 75 und zwei Seitenwände 76 aufweist. Die Einzugseinrichtung 70 ist als ein Strangpressprofil ausgebildet, insbesondere aus Aluminium. Die Einzugseinrichtung 70 ist in Hinblick auf die Grundplatte 75 und die Seitenwände 76 symmetrisch in Bezug auf eine Mittenlängsachse M ausgebildet. An der Mittenlängsachse M, also zentrisch auf der Grundplatte 75, sind zwei Umlenkeinheiten 72 angeordnet, um welche herum ein Zugmittel 71 geführt ist. Das Zugmittel 71 überragt ein erstes Ende 70.1 sowie ein zweites Ende 70.2 der Einzugseinrichtung. Die Einzugseinrichtung weist drei freie Enden auf, nämlich zwei Befestigungseinheiten 71.1 und eine Betätigungseinheit 71.2. Die Befestigungseinheiten 71.1 sind als Schlaufen ausgebildet, welche die Enden 70.1, 70.2 überragen. An diesen Schlaufen 71.1 kann eine Leitung (nicht dargestellt) angekuppelt werden, um die Leitung der Betätigungseinheit 71.2 entlang der Grundplatte 75 zu verlagern. Ferner weist die Einzugseinrichtung 70 einen Kraft- bzw. Energiespeicher 73 auf, insbesondere als elastische Feder ausgebildet. Der Kraftspeicher 73 ist mit dem Zugmittel 71 fest verbunden, insbesondere an einer Art Klemmung oder Pressung.

Beim Betätigen der Betätigungseinheit, die insbesondere als formbeständiger Griff ausgeführt ist, wird nun das Zugmittel 71 verlagert, wodurch der Kraftspeicher 73 vorgespannt wird, so dass eine Rückstellkraft vom Kraftspeicher 73 auf das Zugmittel 71 ausgeübt wird. Wird nun die Betätigungseinheit 71.2 losgelassen, so kann das Zugmittel in eine Ausgangsposition (wie dargestellt) zurückverlagert werden. Auf diese Weise können mehrere Leitungen nacheinander entlang der Einzugseinrichtung 70 verlagert werden. Im gezeigten Ausführungsbeispiel ist der Kraftspeicher als Konstantkraftfeder, insbesondere Spiralfeder, ausgebildet. Der Kraftspeicher 73 ist um eine Achse herumgewickelt, und beim Vorspannen des Kraftspeichers 73 werden die Wicklungen um die Achse herum enger gezogen, wodurch die Vorspannkraft hervorgerufen wird. Die Umlenkeinheiten 72 können als drehbare Rolle, also als um eine Achse gelagerte Trommel oder dergleichen ausgebildet sein, oder auch jeweils als feststehender Bolzen. Bevorzugt weisen die Umlenkeinheiten eine konkave (nach innen gewölbte) Außenmantelfläche auf, welche sicherstellen kann, dass das Zugmittel 71 nicht von der Umlenkeinheit 72 herunterrutschen kann. Dies kann auch durch eine konstante Zugkraft auf das Zugmittel 71 sichergestellt werden, wobei die Zugkraft vom Kraftspeicher 73 ausgeübt werden kann, und zwar auch bereits in der gezeigten Ausgangsposition des Zugmittels 71. Hierdurch kann in jeder Position des Zugmittels eine Zugkraft auf das Zugmittel ausgeübt werden, wodurch auch sichergestellt werden kann, dass die Betätigungseinheit 71.2 wieder zurück in eine Ausgangslage verlagert wird (Rückstellmechanismus).

Wahlweise kann das Zugmittel 70 auch mit einer weiteren Betätigungseinheit (nicht dargestellt) verbunden sein, welche gegenüberliegend zur dargestellten Betätigungseinheit 71.2 im Bereich der gegenüberliegenden Umlenkeinheit 72 angeordnet ist. Hierdurch kann eine Einzugseinrichtung bereitgestellt werden, welche von beiden Seiten 70.1, 70.2 betätigt werden kann.

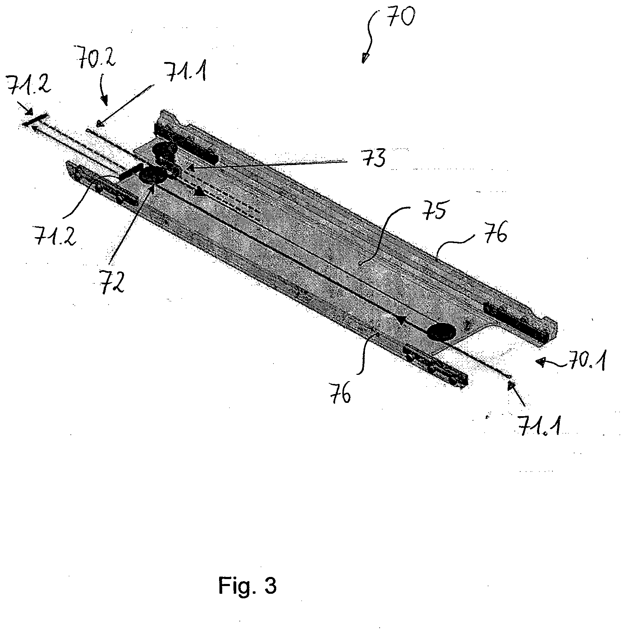

In der Figur 3 ist die Einzugseinrichtung 70 aus einer weiteren Perspektive gezeigt. Mittels Pfeilen und Strichlinien sind dabei die Verlagerungsrichtungen und unterschiedlichen Positionen angedeutet, insbesondere eine verlagerte Position der Betätigungseinheit 71.2. In der Ausgangslage ist die Betätigungseinheit 71.2 im Bereich der einen der Umlenkeinheiten 72 angeordnet, und in der verlagerten Position in einem bedeutenden Abstand zur entsprechenden Umlenkeinheit 72, wobei dann die Befestigungseinheiten 71.1 jeweils nach innen hin zur Mitte der Einzugseinrichtung 70 verlagert sind, d.h. zwei der drei freien Enden der Einzugseinrichtung (nämlich die Befestigungseinheiten 71.1) sind irgendwo entlang der Grundplatte 75 angeordnet, und das dritte freie Ende (die Betätigungseinheit 71.2) ist in einem Abstand zur Grundplatte 75 angeordnet. Im Gegensatz dazu sind in der Ausgangslage die beiden Kupplungen bzw. Befestigungseinheiten 71.1 in einem Abstand zur Grundplatte 75 angeordnet, und die Betätigungseinheit 71.2 ist direkt neben einer der Umlenkeinheiten 72 angeordnet.

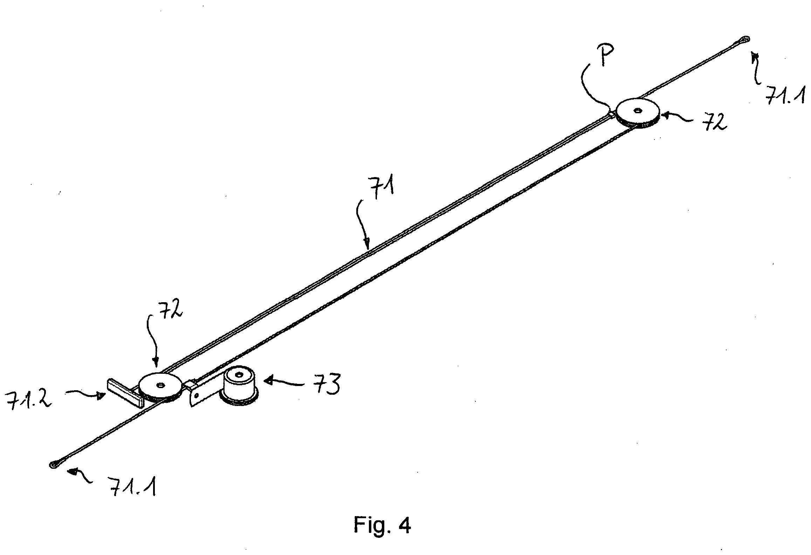

In der Figur 4 ist das Zugmittel 71 mit den Umlenkeinheiten 72 gezeigt. Die Befestigungseinheiten 71.1 sind jeweils an einem Befestigungspunkt P (welcher nur für eine der Befestigungseinheiten 71.1 dargestellt ist) mit dem Zugmittel 71 verbunden. Das Zugmittel 71 ist als ein umlaufendes Seil ausgebildet, an welchem die beiden Befestigungseinheiten 71.1 und die Betätigungseinheit 71.2 befestigt sind. Die Betätigungseinheit 71.2 und eine der Befestigungseinheiten 71.1 können in demselben Befestigungspunkt P mit dem Zugmittel 71 verbunden sein. Ferner können die andere Befestigungseinheit 71.1 und der Kraftspeicher 73 ebenfalls zusammen in einem Befestigungspunkt mit dem Zugmittel 71 verbunden sein. Dies ermöglicht einen einfachen Aufbau.

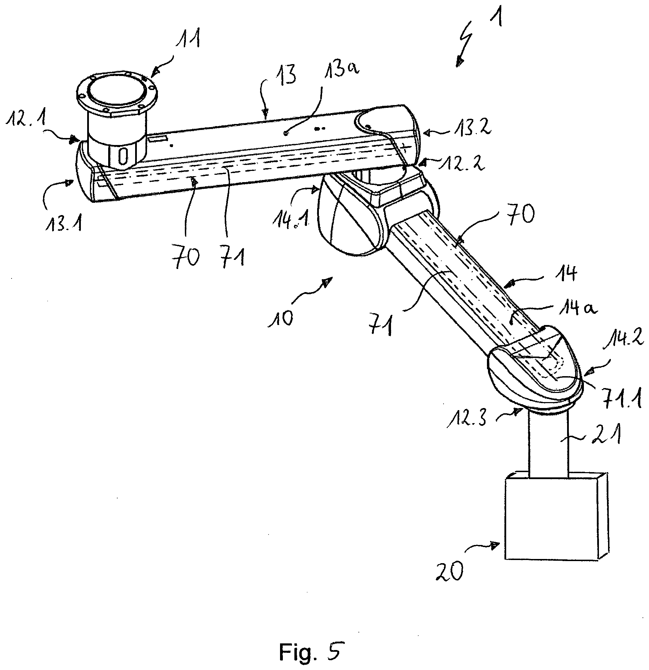

In der Figur 5 ist eine Stativvorrichtung 1 gezeigt, welche einen ersten Tragarm 13 und einen zweiten Tragarm 14 aufweist, wobei am zweiten Tragarm 14 eine Konsole 20 in einem (dritten) Drehgelenk 12.3 gelagert ist. Der erste Tragarm 13 weist eine Oberseite 13a auf, an welcher eine Einzugseinrichtung 70 befestigt ist. Die Einzugseinrichtung 70 erstreckt sich von einem ersten Ende 13.1 des Tragarms bis zu einem zweiten Ende 13.2. Auf dieselbe Weise ist eine weitere Einzugseinrichtung 70 am zweiten Tragarm 14 angeordnet. Die weitere Einzugseinrichtung 70 ist ebenfalls an einer Oberseite 14a des zweiten Tragarms 14 befestigt. Bei diesem Ausführungsbeispiel weist jeder Tragarm 13, 13 eine eigene Einzugseinrichtung 70 auf, so dass eine Leitung (nicht dargestellt) vom Deckenflansch 11 bis hin zur Konsole 20 oder in umgekehrter Richtung komplett innerhalb vom Tragarmsystem 10 verlegt werden kann.

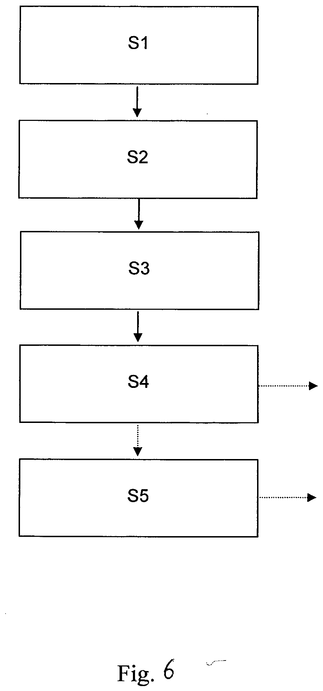

In der Figur 6 ist ein Verfahren zum Verlegen einer Leitung an einer Stativvorrichtung gezeigt, welches die folgenden Schritte umfasst. In einem ersten Schritt S1 erfolgt ein Befestigen einer Einzugseinrichtung an der Stativvorrichtung. Dies kann z.B. durch Verschrauben einer Grundplatte oder Seitenwänden mit einem Tragarm der Stativvorrichtung verfolgen. Es können auch andere Arten der Befestigung vorgesehen sein, z.B. Verkleben, Verklemmen oder Einsetzen in eine Gegenform bzw. entsprechende Aufnahme. In einem zweiten Schritt S2 erfolgt ein Befestigen einer Leitung an einem Zugmittel der Einzugseinrichtung, insbesondere an einer von zwei Befestigungseinheiten. Hierzu kann eine Kupplung der Leitung z.B. in eine durch das Zugmittel gebildete Schlaufe gesteckt werden. In einem dritten Schritt S3 erfolgt ein Ausüben einer Zugkraft auf das Zugmittel zum Verlagern der Leitung mittels des Zugmittels relativ zur Stativvorrichtung, wobei das Zugmittel von einer Ausgangsposition verlagert wird. In einem vierten Schritt S4 erfolgt ein Einziehen der Leitung in den Tragarm mittels des Zugmittels. Der vierte Schritt S4 geht einher mit dem dritten Schritt S3, ist also durch den dritten Schritt S3 bedingt. Im vierten Schritt S4 kann ein freies Ende des Zugmittels manuell ergriffen werden, um eine Zugkraft auf die Leitung auszuüben. Wahlweise kann das Verfahren ferner den folgenden fünften Schritt S5 umfassen: Zurückverlagern des Zugmittels in die Ausgangsposition, insbesondere mittels eines Rückstellmechanismus. Der Rückstellmechanismus kann z.B. im Wesentlichen durch einen Kraftspeicher in Form einer elastischen Feder gebildet sein. Nachdem das Zugmittel in die Ausgangsposition zurückverlagert wurde, ist die Befestigungseinheit bzw. sind die Befestigungseinheiten wieder zugänglich, und es kann eine weitere Leitung an die Befestigungseinheit(en) gekuppelt werden. Dank des Rückstellmechanismus ist es nicht erforderlich, irgendeinen Draht durch den jeweiligen Tragarm hindurchzufädeln. Die Befestigungseinheiten können automatisch zurückgezogen werden, um die Befestigungseinheiten wieder in eine (manuell) zugängliche Ausgangslage zu bringen.

### Bezugszeichenliste

- 1: Stativvorrichtung
- 10: Tragsystem
- 11: Montageeinrichtung, insbesondere Deckenflansch
- 12.1: (erstes) Drehgelenk
- 12.2: zweites Drehgelenk
- 12.3: drittes Drehgelenk
- 13: (erster) Tragarm
- 13a: Oberseite des ersten Tragarms
- 13.1: erstes Ende des ersten Tragarms
- 13.2: zweites Ende des ersten Tragarms
- 14: zweiter Tragarm
- 14a: Oberseite des zweiten Tragarms
- 14.1: erstes Ende des zweiten Tragarms
- 14.2: zweites Ende des zweiten Tragarms

- 20: medizintechnische Einrichtung, insbesondere Versorgungskonsole
- 21: Träger, insbesondere Konsolenrohr
- 22: Griff
- 23: Taste

- 70: Einzugseinrichtung
- 70.1: erstes Ende der Einzugseinrichtung
- 70.2: zweites Ende der Einzugseinrichtung
- 71: Zugmittel, insbesondere Seil, z.B. Stahlseil
- 71.1: Befestigungseinheit, insbesondere Schlaufe
- 71.2: Betätigungseinheit, insbesondere formbeständiger Griff
- 72: Umlenkeinheit, insbesondere drehbare Rolle oder feststehender Bolzen
- 73: Kraftspeicher oder Energiespeicher, insbesondere elastische Feder
- 75: Grundplatte
- 76: Seitenwand
- M: Mittenlängsachse der Einzugseinrichtung

- P: Befestigungspunkt

- S1: erster Schritt
- S2: zweiter Schritt
- S3: dritter Schritt
- S4: vierter Schritt
- S5: fünfter Schritt

## Patentansprüche

1. Stativvorrichtung (1) zur Anordnung in einem Operationssaal, umfassend
- ein Tragsystem (10) umfassend eine Montageeinrichtung (11) und mindestens einen Tragarm (13, 14);
- eine medizintechnische Einrichtung (20), die am mindestens einen Tragarm befestigt ist;
**dadurch gekennzeichnet, dass** die Stativvorrichtung (1) eine Einzugseinrichtung (70) aufweist, die mit einer Leitung verbindbar ist und eingerichtet ist, die Leitung in die Stativvorrichtung (1), insbesondere in den mindestens einen Tragarm, nachträglich einzuziehen und zu verlegen, wobei die Einzugseinrichtung eingerichtet ist, die Leitung in einer ersten Richtung oder in einer zweiten Richtung entgegengesetzt zur ersten Richtung einzuziehen und zu verlegen.

2. Stativvorrichtung nach Anspruch 1, **dadurch gekennzeichnet, dass** die Einzugseinrichtung (70) am mindestens einen Tragarm (13, 14) befestigt ist.

3. Stativvorrichtung nach Anspruch 1 oder 2, **dadurch gekennzeichnet, dass** die Einzugseinrichtung (70) innerhalb vom mindestens einen Tragarm angeordnet ist, insbesondere an einer Oberseite (13a, 14a) des Tragarms.

4. Stativvorrichtung nach einem der vorhergehenden Ansprüche, **dadurch gekennzeichnet, dass** die Einzugseinrichtung eine Platte (75) aufweist, insbesondere ein metallisches Strangpressprofil oder Kunststoffteil.

5. Stativvorrichtung nach einem der vorhergehenden Ansprüche, **dadurch gekennzeichnet, dass** die Einzugseinrichtung sich entlang des mindestens einen Tragarms (13, 14) erstreckt und derart daran angeordnet ist, dass die Einzugseinrichtung sowohl von einem ersten Ende des Tragarms (13.1, 14.1) als auch von einem zweiten Ende (13.2, 14.2) des Tragarms für eine manuelle Betätigung der Einzugseinrichtung zugänglich ist.

6. Stativvorrichtung nach einem der vorhergehenden Ansprüche, **dadurch gekennzeichnet, dass** die Stativvorrichtung eine Mehrzahl von Tragarmen (13, 14) aufweist, wobei innerhalb von mindestens zwei der Tragarme (13, 14) jeweils eine Einzugseinrichtung (70) angeordnet ist.

7. Stativvorrichtung nach einem der vorhergehenden Ansprüche, **dadurch gekennzeichnet, dass** die Einzugseinrichtung ein Zugmittel (71) zum Aufbringen einer Zugkraft auf die Leitung aufweist.

8. Stativvorrichtung nach Anspruch 7, **dadurch gekennzeichnet, dass** das Zugmittel (71) mindestens eine manuelle Betätigungseinheit (71.2) aufweist, insbesondere einen Griff.

9. Stativvorrichtung nach einem der vorhergehenden Ansprüche, **dadurch gekennzeichnet, dass** die Einzugseinrichtung mindestens einen Rückstellmechanismus (73), insbesondere einen Kraftspeicher aufweist.

10. Stativvorrichtung nach einem der vorhergehenden Ansprüche, **dadurch gekennzeichnet, dass** die Einzugseinrichtung mindestens eine Umlenkeinheit (72), insbesondere Umlenkrolle umfasst.

11. Stativvorrichtung nach einem der vorhergehenden Ansprüche, **dadurch gekennzeichnet, dass** die Einzugseinrichtung eine Befestigungseinheit (71.1) zum Befestigen der Leitung an der Einzugseinrichtung aufweist.

12. Stativvorrichtung nach einem der vorhergehenden Ansprüche, **dadurch gekennzeichnet, dass** die Einzugseinrichtung eine manuelle Betätigungseinheit (71.2) aufweist, welche an einem Zugmittel (71) der Einzugseinrichtung fixiert ist, wobei das Zugmittel zwei Befestigungseinheiten (71.1) bereitstellt, von denen die eine Befestigungseinheit von einem ersten Ende (70.1) der Einzugseinrichtung hervorsteht und von denen die andere Befestigungseinheit von einem zweiten Ende (70.2) der Einzugseinrichtung hervorsteht.

13. Einzugseinrichtung (70) für eine Stativvorrichtung (1) nach einem der vorhergehenden Ansprüche, **dadurch gekennzeichnet, dass** die Einzugseinrichtung eine Platte (75) sowie ein relativ zur Platte verlagerbares und mit der Platte verbundenes Zugmittel (71) aufweist, welches mit einer Leitung zum Einziehen der Leitung in die Stativvorrichtung verbindbar ist.

14. Verfahren zum Verlegen einer Leitung an einer in einem Operationssaal angeordneten Stativvorrichtung (1) nach einem der vorhergehenden Ansprüche, **gekennzeichnet durch** die Schritte:
- Befestigen einer Einzugseinrichtung (70) an der Stativvorrichtung;
- Befestigen einer Leitung an einem Zugmittel (71) der Einzugseinrichtung;
- Ausüben einer Zugkraft auf das Zugmittel (71) zum Verlagern der Leitung mittels des Zugmittels (71) relativ zur Stativvorrichtung, wobei das Zugmittel von einer Ausgangsposition verlagert wird; und dadurch
- Einziehen der Leitung in die Stativvorrichtung mittels des Zugmittels.

## Claims

1. A stand device (1) for placement in an operating theatre, comprising
- a support system (10) comprising a mounting device (11) and at least one support arm (13, 14);
- a medical device (20) which is attached to at least one support arm;
**characterized in that** the stand device (1) comprises a draw-in device (70) connectable to a cable and arranged to subsequently draw-in and lay the line in the stand device (1), in particular in the at least one support arm, the draw-in device being arranged to draw in and lay the line in a first direction or in a second direction opposite to the first direction.

2. Stand device according to Claim 1, **characterized in that** the draw-in device (70) is mounted on at least one support arm (13, 14).

3. Stand device according to claim 1 or 2, **characterized in that** the draw-in device (70) is arranged inside the at least one support arm, in particular on an upper side (13a, 14a) of the support arm.

4. Stand device according to one of the preceding claims, **characterized in that** the draw-in device comprises a plate (75), in particular a metallic extruded profile or plastic part.

5. Stand device according to one of the preceding claims, **characterized in that** the draw-in device extends along the at least one support arm (13, 14) and is arranged thereon such that the draw-in device is accessible both from a first end of the support arm (13.1, 14.1) and from a second end (13.2, 14.2) of the support arm for manual operation of the draw-in device.

6. Stand device according to one of the preceding claims, **characterized in that** the stand device has a plurality of support arms (13, 14), a respective draw-in device (70) being arranged inside at least two of the support arms (13, 14).

7. A stand device according to one of the preceding claims, **characterized in that** the draw-in device comprises pulling means (71) for applying a pulling force to the cable.

8. Stand device according to claim 7, **characterized in that** the pulling means (71) comprises at least one manual operating unit (71.2), in particular a handle.

9. Stand device according to one of the preceding claims, **characterized in that** the draw-in device has at least one resetting mechanism (73), in particular an energy accumulator.

10. Stand device according to one of the preceding claims, **characterized in that** the draw-in device comprises at least one deflection unit (72), in particular a deflection roller.

11. Stand device according to one of the preceding claims, **characterized in that** the draw-in device comprises a fixing unit (71.1) for fixing the line to the draw-in device.

12. A stand device according to one of the preceding claims, **characterized in that** the draw-in device comprises a manual operating unit (71.2) fixed to a pulling means (71) of the draw-in device, the pulling means providing two fastening units (71.1), one fastening unit projecting from a first end (70.1) of the draw-in device and the other fastening unit projecting from a second end (70.2) of the draw-in device.

13. A draw-in device (70) for a stand device (1) according to one of the preceding claims, **characterized in that** the draw-in device comprises a plate (75) and a pulling means (71) displaceable relative to the plate and connected to the plate, which is connectable to a line for drawing the line into the stand device.

14. Method for laying a line on a stand device (1) arranged in an operating theatre according to one of the preceding claims, **characterized by** the steps:
- mounting a draw-in device (70) on the stand device;
- fastening a line to a pulling means (71) of the draw-in device;
- exerting a pulling force on said pulling means (71) to displace said line by said pulling means (71) relative to said stand device, said pulling means being displaced from an initial position; and thereby
- pulling the line into the stand device by means of the pulling device.

## Revendications

1. Dispositif de support (1) destiné à être placé dans une salle d'opération, comprenant
- un système porteur (10) comprenant un dispositif de montage (11) et au moins un bras porteur (13, 14) ;
- un dispositif médical (20) qui est fixé au au moins un bras porteur;
**caractérisé en ce que** le dispositif de support (1) comprend un dispositif d'insertion (70) pouvant être relié à une ligne et agencé pour après tirer et poser la ligne dans le dispositif de support (1), en particulier dans le au moins un bras porteur, le dispositif d'insertion étant agencé pour tirer et poser la ligne dans une première direction ou dans une seconde direction opposée à la première direction.

2. Dispositif de support selon la revendication 1, **caractérisé en ce que** le dispositif d'insertion (70) est fixé au au moins un bras porteur (13, 14).

3. Dispositif de support selon la revendication 1 ou 2, **caractérisé en ce que** le dispositif d'insertion (70) est disposé à l'intérieur du au moins un bras porteur, en particulier sur un côté supérieur (13a, 14a) du bras porteur.

4. Dispositif de support selon l'une des revendications précédentes, **caractérisé en ce que** le dispositif d'insertion comprend une plaque (75), en particulier un profilé métallique extrudé ou une pièce plastique.

5. Dispositif de support selon l'une des revendications précédentes, **caractérisé en ce que** le dispositif d'insertion s'étend le long d'au moins un bras porteur (13, 14) et est disposé sur celui-ci de telle sorte que le dispositif d'insertion est accessible à la fois depuis une première extrémité du bras porteur (13.1, 14.1) et depuis une seconde extrémité (13.2, 14.2) du bras porteur pour une opération manuelle du dispositif d'insertion.

6. Dispositif de support selon l'une des revendications précédentes, **caractérisé en ce que** le dispositif de support comporte plusieurs bras porteur (13, 14), un dispositif d'insertion (70) respectif étant disposé à l'intérieur d'au moins deux des bras porteur (13, 14).

7. Dispositif de support selon l'une des revendications précédentes, **caractérisé en ce que** le dispositif d'insertion comprend des moyens de traction (71) pour appliquer une force de traction à la ligne.

8. Dispositif de support selon la revendication 7, **caractérisé en ce que** le moyen de traction (71) comprend au moins une unité de commande manuelle (71.2), en particulier une poignée.

9. Dispositif de support selon l'une des revendications précédentes, **caractérisé en ce que** le dispositif d'insertion présente au moins un mécanisme de rappel (73), en particulier un accumulateur d'énergie.

10. Dispositif de support selon l'une des revendications précédentes, **caractérisé en ce que** le dispositif d'insertion comprend au moins une unité de déviation (72), en particulier un rouleau de déviation.

11. Dispositif de support selon l'une des revendications précédentes, **caractérisé en ce que** le dispositif d'insertion comprend une unité de fixation (71.1) pour fixer la ligne sur le dispositif d'insertion.

12. Dispositif de support selon l'une des revendications précédentes, **caractérisé en ce que** le dispositif d' insertion comprend une unité de commande manuelle (71.2) fixée à un moyen de traction (71) du dispositif d'insertion, le moyen de traction fournissant deux unités de fixation (71.1), une unité de fixation dépassant d'une première extrémité (70.1) du dispositif d'insertion et l'autre unité de fixation dépassant d'une seconde extrémité (70.2) du dispositif d'insertion.

13. Dispositif d'insertion (70) pour un dispositif de support (1) selon l'une des revendications précédentes, **caractérisé en ce que** le dispositif d'insertion comprend une plaque (75) et un moyen de traction (71) déplaçable par rapport à la plaque et relié à la plaque et pouvant être relié à un conduit pour introduire la ligne dans le dispositif de support.

14. Procédé de pose d'une ligne sur un dispositif de support (1) disposé dans une salle d'opération selon l'une des revendications précédentes, **caractérisé par** les étapes suivantes :
- fixation d'un dispositif d'insertion (70) sur le dispositif de support;
- fixation d'une ligne à un moyen de traction (71) du dispositif d'insertion;
- exercer une force de traction sur ledit moyen de traction (71) pour déplacer ladite ligne par ledit moyen de traction (71) par rapport audit dispositif de support, ledit moyen de traction étant déplacé depuis une position initiale; et ainsi
- tirer la ligne dans le dispositif de support à l'aide du dispositif de traction.
